(19) Europäisches Patentamt
European Patent Office
Office européen des brevets

(11) Publication number: **0 479 166 A1**

# (12) EUROPEAN PATENT APPLICATION

(21) Application number: **91116588.4**

(22) Date of filing: **27.09.91**

(51) Int. Cl.[5]: **C07D 301/00**, C07D 303/40, C08G 63/91, //C08G59/20

(30) Priority: **28.09.90 US 591455**

(43) Date of publication of application: **08.04.92 Bulletin 92/15**

(84) Designated Contracting States: **AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Stanley, James Preston**
**1780 Saddlebrook Road**
**Bound Brook, New Jersey 08805(US)**
Inventor: **Upshaw, Thomas Andrew**
**70 JFK Boulevard, Apt. 26K**
**Somerset, New Jersey 08873(US)**

(74) Representative: **von Hellfeld, Axel, Dipl.-Phys. Dr. et al**
**Wuesthoff & Wuesthoff Patent- und Rechtsanwälte Schweigerstrasse 2**
**W-8000 München 90(DE)**

## (54) Transesterification process for preparation of cycloaliphatic epoxides.

(57) Transesterification and a particular class of catalysts, namely, one or more salts of one or more weak acids, and mixtures thereof, are used to transesterify epoxide esters and active hydrogen-containing compounds to produce a wide variety of cycloaliphatic epoxide products.

EP 0 479 166 A1

## BACKGROUND OF THE INVENTION

### Field of the Invention

This invention relates to a process for the manufacture of novel monofunctional and multifunctional liquid or solid cycloaliphatic epoxides that are useful in a variety of applications. The process involves transesterification of lower alkyl cycloaliphatic epoxide esters and suitable active-hydrogen compounds such as alcohols, glycols, polyols, and the like in the presence of a suitable catalyst to produce a variety of cycloaliphatic epoxides.

### Description of the Prior Art

Cycloaliphatic epoxides corresponding to the structural formula,

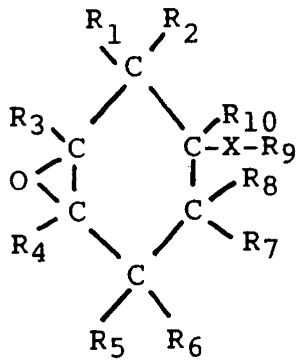

wherein $R_1$ through $R_8$ are the same or different and are hydrogen, phenyl, or unsubstituted or substituted lower alkyl groups, $R_9$ is linear or branched lower alkyl, X is COO or $COOCH_2CH(R_{11})O$, and $R_{10}$ and $R_{11}$ are the same or different and are hydrogen or methyl, as well as similar compounds are described in copending, co-assigned patent application of Koleske et al. entitled Novel Unsaturated Cyclic Carboxylates and Related Compounds, Serial Number _______, filed on _______ [Attny Dkt. D-16035], the disclosure of which is incorporated herein by reference.

In addition, U. S. Patent 3,674,793 mentions the use of lower-alkyl 3,4-epoxycyclohexanecarboxylates as intermediates in the preparation of diones. In this citation, the term "lower alkyl" meant an alkyl radical having from one to six carbon atoms inclusive and was illustrated by methyl, ethyl, isopropyl, n-propyl, n-butyl, sec-butyl, isobutyl, n-amyl, n-hexyl, and the like.

Also, Klunder and coworkers, J. Org. Chem., 50, 912-915 (1985) have described the stereoselectivity of a particular reagent system in the chlorohydroxylation of olefins. Various epoxides including methyl 3,4-epoxycyclohexanecarboxylate are illustrated for comparison purposes.

Batog and coworkers describe the reaction of ethyl 3,4-epoxycyclohexanecarboxylate with carboxylic acids in Zh. Org. Khim. 16 (6), 1126 (1980), and the kinetics of epoxidation of methyl 3-cyclohexene carboxylate in Zh. Org. Khim. 17, 2085 (1981).

The process of transesterification in which ester-interchange reactions occur is also known. An apparatus and experimental method for the process of transesterification has been described by E. Tucek and H.-D. Dinse, Acta Polymerica, 31 (7), 429 (1980). Catalysts that have been mentioned as useful for promoting the transesterification process include compounds such as tetrabutyl titanate, zinc acetate, titanium dioxide, sodium and potassium alkoxides, sodium and potassium phenoxides, lead oxide, ion exchange resins, and the like.

U.S. Patents 3,454,531 and 3,457,238 mention the use of barium, calcium, cobalt, lead, lithium, sodium, and zinc thiocyanates as transesterification catalysts for the preparation of poly(ethylene terephthalate). Potassium thiocyanate is not mentioned in these references. Various investigators, Bradshaw, J. S. and coworkers, J. Heterocyclic Chem., 20, 353 (1983) and Nakatsuji, Y. and J. S. Bradshaw, Croatica Chem. Acta., 59, 19 (1986) have used metal methoxides in the presence and absence of molecular sieves to produce macrocyclic diesters by transesterification of methyl esters.

U. S. Patent 4,228,084 is concerned with the preparation of glycidyl methacrylate by transesterification of methyl methacrylate with glycidol in the presence of a polymerization inhibitor and a transesterification catalyst. The catalyst is a salt of the form MX where M is an alkali metal ion and X is a cyanide, cyanate, or

thiocyanate ion.

U.S. Patent 3,048,601 is concerned with transesterification of methyl and ethyl 2,3-epoxyalkanoates with allyl alcohol in the presence of metal alcoholate catalysts such as sodium and potassium methoxide and as sodium and potassium allyloxide.

In above-cited copending, co-assigned patent application Novel Unsaturated Cyclic Carboxylates and Related Compounds, Serial Number _______, filed on _______, [Attny Dkt. D-16035] a variety of processes is described. That patent application discloses the use of transesterification as a technique to prepare numerous diverse compounds from cyclohexene compounds. However, it further points out that if the technique of transesterification is to be used with cycloaliphatic epoxides, the epoxides may undergo a ring-opening reaction during the transesterification reaction. Such a ring-opening reaction is undesirable since the epoxide functionality would be lost.

## SUMMARY OF THE INVENTION

It has been discovered that the process of transesterification of cycloaliphatic epoxides with active hydrogen compounds can be successfully carried out if certain salts of weak acids are employed as catalysts. Certain cycloaliphatic epoxides produced by the process are novel compositions and can be either solid or liquid as well as monofunctional or multifunctional in nature and, in certain instances, can be polymeric in nature.

Cycloaliphatic epoxides useful in the process are those corresponding to the structural formula:

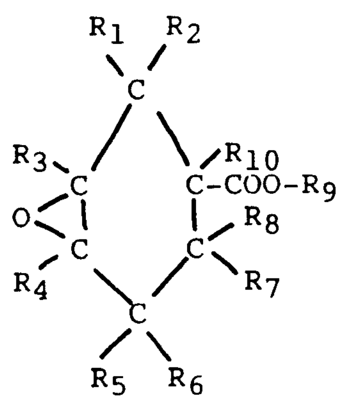

wherein $R_1$ through $R_7$ are the same or different and are hydrogen, phenyl, halogen, or unsubstituted or substituted lower alkyl groups of one to about six carbon atoms with it preferred that at least one of $R_3$ or $R_4$ be hydrogen, $R_8$ is hydrogen, phenyl, halogen, $-COOR_9'$, or unsubstituted or substituted lower alkyl groups of one to about six carbon atoms, $R_9'$ is lower alkyl of one to about six carbon atoms with it preferred that $R_9'$ is one to four carbon atoms, $R_9$ is the same or different from $R_9'$ and is lower alkyl of one to about six carbon atoms with it preferred that $R_9$ is one to four carbon atoms, and $R_{10}$ is hydrogen or methyl. It is most preferred that $R_1$, $R_2$, $R_5$, $R_6$, and $R_7$ as well as one of $R_3$ and $R_4$ be hydrogen, and that the other of $R_3$ and $R_4$ be methyl, that $R_8$ be hydrogen or $-COOR_9'$, that $R_9$ and $R_9'$ be one to four carbon atoms, and $R_{10}$ be hydrogen or methyl.

Although many of the products of the invention can be produced by other techniques, such as by transesterification of lower-alkyl cyclohexenecarboxylates with compounds containing hydroxyl functionality, followed by epoxidation and by condensation of cyclohexene carboxylates or epoxycyclohexanecarboxylates with hydroxyl functional compounds, transesterification of lower-alkyl epoxycyclohexanecarboxylates with compounds containing hydroxyl functionality has a number of advantages. These advantages include the following:

1. The process allows use of a distilled product, a lower-alkyl epoxycyclohexanecarboxylate, to produce the final compounds and thus eliminates oligomer formation and accompanying viscosity increases that usually occur during the epoxidation process.

2. In addition to eliminating oligomers in the final product, the process also eliminates other impurities that arise during epoxidation, such as carboxylic acids and anions or cations that can be detrimental to shelf life, electrical properties, or other properties.

3. Only one epoxidation process needs to be developed and optimized, since transesterification allows a single product to be used for the manufacture of numerous other products.

4. Since epoxidation processes are more difficult to conduct and optimize with high yields in comparison to transesterification processes, the transesterification process allows for improved flexibility in manufacturing facility design requirements and in making designs more ecologically sound with a strong potential for saving valuable resources.

The cycloaliphatic epoxides produced by the process of the invention are useful as acid scavengers; as intermediates for manufacture of molded parts; as components of coatings, inks, sealants, and adhesives cured by either thermal and/or radiation techniques; as crosslinking agents for powder coatings, pharmaceutical products or intermediates for pharmaceutical products; as perfumes and fragrances; as photoresists or components of photoresists of various types for printed circuit boards and semiconductor chips; as reactive solvents when they have a low viscosity; as powder coating materials when solid in nature; as intermediates for the production of higher functionality epoxides; as well as in other end uses.

## DETAILED DESCRIPTION OF THE INVENTION

In the novel process and method for carrying out this invention, a transesterification process employing particular catalysts is used to manufacture cycloaliphatic epoxides from lower-alkyl epoxycyclohexanecarboxylates and active hydrogen-containing compounds such as alcohols, glycols, polyols, and polymeric materials.

Illustrative of the cycloaliphatic epoxides useful in the process of the invention are methyl 3,4-epoxycyclohexane-carboxylate, ethyl 3,4-epoxycyclohexanecarboxylate, propyl 3,4-epoxycyclohexanecarboxylate, isopropyl 3,4-epoxycyclohexanecarboxylate; n-butyl-, i-butyl-, s-butyl-, and t-butyl 3,4-epoxycyclohexanecarboxylate; the various amyl and the various hexyl 3,4-epoxycyclohexanecarboxylates which have the form:

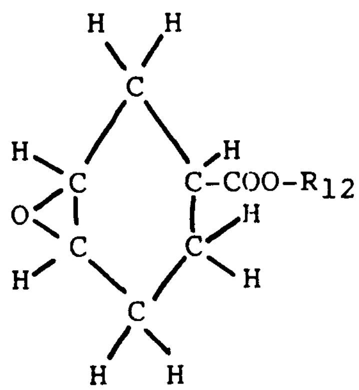

wherein $R_{12}$ is linear or branched $C_5H_{11}$ or $C_6H_{13}$; methyl 3,4-epoxy-3-methyl-cyclohexanecarboxylate; ethyl 3,4-epoxy-3-methyl-cyclohexanecarboxylate; methyl 3,4-epoxy-4-methyl-cyclohexanecarboxylate; ethyl 3,4-epoxy-4-methyl-cyclohexanecarboxylate; butyl 3,4-epoxy-3-methyl-cyclohexanecarboxylate; butyl 3,4-epoxy-4-methyl-cyclohexanecarboxylate; dialkyl 4,5-epoxycyclohexane-1,2-dicarboxylates of the form

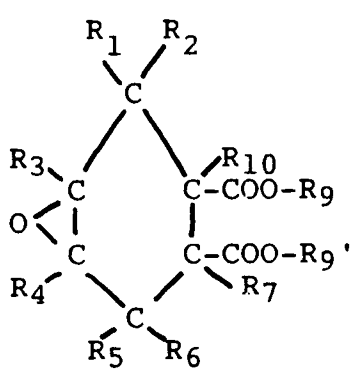

illustrative of which are dimethyl 4,5-epoxycyclohexane-1,2-dicarboxylate, diethyl 4,5-epoxycyclohexane-1,2-dicarboxylate, the dipropyl 4,5-epoxycyclohexane-1,2-dicarboxylates, the dibutyl 4,5-epoxycyclohexane-1,2-dicarboxylates, the diamyl 4,5-epoxycyclohexane-1,2-dicarboxylates, the dihexyl 4,5-epoxycyclohexane-1,2-dicarboxylates, as well as mixed dialkyl 4,5-epoxycyclohexane-1,2-dicarboxylates, such as 1-methyl,2- ethyl 4,5-epoxycyclohexane-1,2- dicarboxylate, 1-ethyl,2-methyl 4,5-epoxycyclohexane-1,2-dicarboxylate, 1-butyl,2- ethyl 4,5-epoxycyclohexane-1,2-dicarboxylate, 1-hexyl,2- methyl 4,5-epoxycyclohexane-1,2- dicarboxylate, 1-ethyl,2-butyl 4,5-epoxy-cyclohexane-1,2-dicarboxylate, 1-propyl,2- amyl 4,5-epoxycyclohexane-1,2-dicarboxylate; and the like.

The active-hydrogen compounds useful in the process of this invention include alcohols, glycols, polyols, and oligomeric and polymeric compounds that contain hydroxyl groups, as well as mixtures of these active-hydrogen compounds, with the understanding that monofunctional or difunctional active-hydrogen compounds are particularly useful when $R_8$ is $-COOR_9'$. Illustrative of the alcohols are the propanols, butanols, pentanols, hexanols, heptanols, octanols, nonanols, decanols, as well as higher alcohols including hydroxyl-containing polyethylenes; 2'-, 3'-, and 4'-hydroxy-acetophenone; 3-cyclohexene-1-methanol; 3,4- epoxycyclohexane-1-methanol; 3,4-epoxy-3-methyl-cyclohexane-1-methanol; 3,4-epoxycyclohexane-1-hydroxyethyl esters of the form:

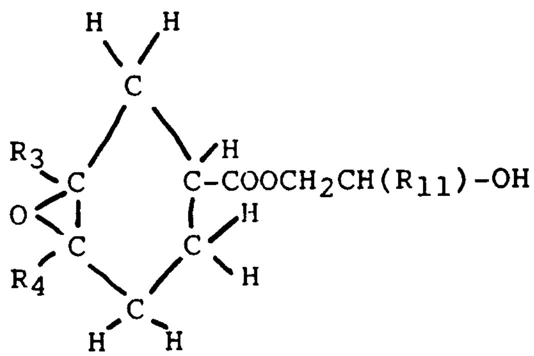

;

2-methanol-dihydropyran; 2-methanol pyran; 1-(hydroxyallyl)trimethylsilane;3α- and 3β-hydroxy-5β-androstan-17-one as well as other androstanolones; hydroxyanisole; 2-, 3-, and 4-hydroxy-benzaldehyde; 2-, 3-, and 4-hydroxybenzophenone; 4-hydroxy-phenylacetonitrile; 1- and 3-hydroxy-2-butanone; 4- and 7-hydroxycoumarin; hydroxyethyl-, hydroxypropyl-, and hydroxylbutyl acrylates, methacrylates, and ethacrylates, including derivatives of these acrylates, methacrylates, and ethacrylates in which they have been reacted with up to about 20 moles of propylene oxide, ethylene oxide, lactones, particularly ε-caprolactone, and/or tetrahydrofuran; tropolone; 2,5-dimethyl-4-hydroxy-3(2H)-furanone, and the like. Illustrative of the glycols are ethylene glycol, diethylene glycol, triethylene glycol, tetraethylene glycol, 1,2- and 1,3-propane diol; 2-methyl-1,3-propanediol; 1,2-, 1,3-, and 1,4-butanediol; 1,3-sec-butanediol; 1,5- and other pentanediols; 1,6 and other hexanediols; 1,10- and other decanediols; neopentyl glycol; 2,2-dimethyl-3-hydroxypropyl 2,2-dimethyl-3-hydroxypropionate, 2,2-dimethyl-4-hydroxybutyl 2,2-dimethyl-3-hydroxypropionate, 2-ethyl-2-butyl-3-hydroxypropyl 2-ethyl-2-butyl-3-hydroxypropionate, and other like esterdiols; propylene glycols; 2-(2-hydroxyethoxy)phenol; 5-cholestene-3β,20α-diol; 3,4-epoxycyclohexane-1,1-dimethanol; 18,21-dihydroxypregn-4-ene-3,20-dione; 3-cyclohexene-1,1-dimethanol; 2-, 3-, and 4-hydroxybenzyl alcohol, and the like. Illustrative of the polyols are glycerol, trimethylolpropane, ditrimethylolpropane, pentaerythritol, dipentaerythritol, tripentaerythritols, trimethylolethane; 1,2,3- and 1,3,4-trihydroxybenzene; 2,3,4- and 2,4,4'-trihydroxybenzophenone; sucrose, dextrose, glucose, sorbitol, fructose, mannose, manitol; ester-,3,5(10)-triene- 2,3,17-triol, galaning, L-erythrulose, 1,2,6- trihydroxyhexane, and the like. Illustrative of the hydroxyl-containing oligomers and polymers are di-, tri-, and higher hydroxyl-functional poly(ethylene glycols), polyoxyethylenes, poly(propylene glycols), poly(oxypropylenes), ethylene oxide-capped and caprolactone- capped propylene oxide polyols that contain up to about 25% by weight ethylene oxide or caprolactone; random, block, and graft ethylene oxide/propylene oxide copolymers; poly-(tetramethylene glycols); the polyester polyols such as hexanediol adipates, diethylene glycol adipates, ethylene glycol adipates, butylene adipates, hexanediol sebacate; 1,4-butanediol/trimethylolpropane adipates; polycarbonate polyols; polycaprolactone polyols; ethylene/vinyl acetate/vinyl alcohol copolymers; ethylene/vinyl alcohol copolymers; vinyl chloride/vinyl acetate/vinyl alcohol copolymers; vinyl chloride/vinyl acetate/hydroxypropyl acrylate copolymers; vinyl chloride/vinyl acetate/hydroxyethyl acrylate copolymers; styrene/allyl alcohol copolymers; styrene/alkyl acrylate/allyl alcohol copolymers; styrene/alkyl acrylate/hydroxyalkyl acrylate copolymers; hydroxyl-containing diglycidyl ethers of Bisphenol A;

hydroxyethyl- and hydroxypropyl cellulose; secondary cellulose acetate, cellulose; adducts prepared by reacting alkylene oxides, lactone, or tetrahydrofuran with the hydroxyl group on alcohols, glycols, polyols, and hydroxyl-containing oligomers and polymers; and the like. If desired, mixtures of two or more active hydrogen compounds can be used in the processes of this invention.

Although an equivalent amount of the lower-alkyl epoxycyclohexanecarboxylates and active hydrogen-containing compound can be used for the transesterification process when an essentially completely transesterified product is desired, to have the process proceed in a rapid, efficient manner, it is preferred that an excess of up to about 5 equivalents of the lower-alkyl epoxycyclohexanecarboxylate per equivalent of active hydrogen compound be used, and most preferred that up to about 2.5 equivalents of the lower-alkyl epoxycyclohexanecarboxylate per equivalent of active hydrogen compound be used. Residual lower alkyl epoxycyclohexanecarboxylate can be allowed to remain in the final product wherein it would act as a reactive diluent, or can be removed by distillation or other suitable separation technique. In a particular embodiment of this invention, less than the equivalent amount of the lower alkyl epoxycyclohexanecarboxylate per equivalent of active hydrogen compound can be used to produce compounds that have both epoxide and residual active hydrogen functionality, as described later in the specification. In this embodiment, from about 0.05 to less than 1.0 equivalent of the lower alkyl epoxycyclohexanecarboxylate per equivalent of active hydrogen compound can be used, or the reaction time and/or temperature can be altered, or sterically-hindered reactants can be used. The resulting compounds with small amounts of epoxide in the final product are particularly useful as acid scavengers before or after attachment by anchoring through the hydroxyl group or groups to other molecules, as intermediates for further manufacture, as dual functional reactants for the purpose of crosslinking, and the like.

Illustrative of the catalysts for the transesterification reaction are sodium bicarbonate, potassium bicarbonate, potassium thiocyanate, barium thiocyanate, calcium thiocyanate, cesium thiocyanate, cobalt thiocyanate, lead thiocyanate, lithium thiocyanate, sodium thiocyanate, zinc thiocyanate, other salts of weak acids such as the sodium acetate, lithium acetate, potassium acetate, cesium acetate, calcium acetate, zinc acetate, and other metal salts of acetic acid and carbonic acid, alkali metal alkoxides such as sodium methoxide, potassium methoxide, lithium methoxide, zinc methoxide, calcium methoxide, cesium methoxide, potassium cyanide, sodium cyanide, metal oxalates, calcium hydride, and the like. In addition, mixtures of the catalysts can be used when desired. The catalysts can be used in amounts of from about 0.10 mol percent to about 25 mol percent, with it preferred that from about 0.25 mol percent to about 20 mol percent be used, and most preferred that from about 0.5 mole percent to about 10 mole percent be used. The catalyst may be add to the reaction mass all at one time, in discrete portions that may be of the same or different size, or in a continuous uniform or nonuniform manner over the entire reaction time period or over a portion of the reaction time period.

The transesterification reaction can be carried out at temperatures of about 50°C to about 250°C and preferably at temperatures of about 70°C to about 200°C. The temperature involved will be dependant on the particular reactants used, the reactant concentration when an inert solvent is used, and particularly on the boiling point of the leaving alcohol. Time for completion of the transesterification reaction will vary from about 10 minutes to 40 or more hours depending on the temperature employed and the particular ingredients involved. The preferred time of reaction is from about one to about eight hours. The transesterification can be carried out under atmospheric pressure, subatmospheric pressure, or superatmospheric pressure; however, it is preferred that the reaction be carried out at pressures of about 0.1 mmHg to about 1 atmosphere. The particular process parameters employed will depend on the particular ingredients used, as well as the batch size.

The transesterification reaction may be depicted by the following reaction scheme when $R_8$ is hydrogen, phenyl, halogen, or substituted or unsubstituted lower alkyl groups:

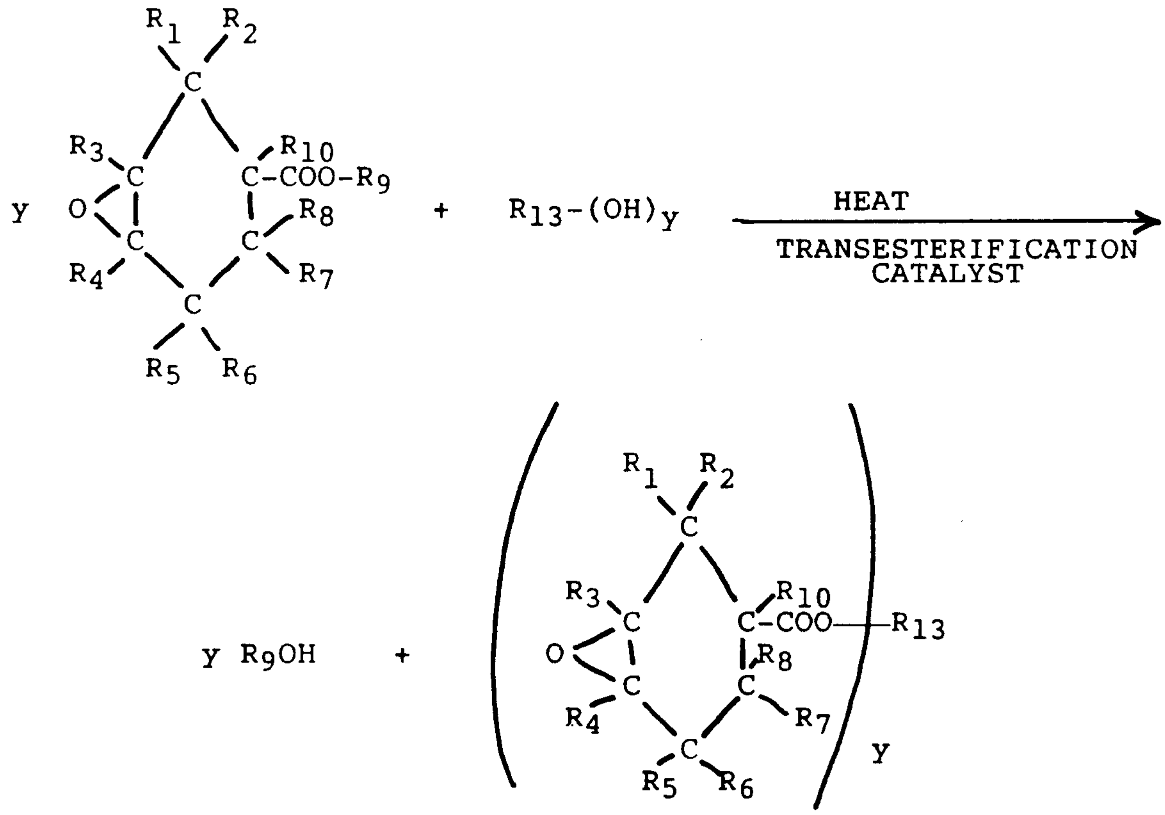


wherein $R_{13}$ is the residue of the active-hydrogen compound-- alcohol, glycol, polyol, oligomeric or polymeric compound that contains hydroxyl groups--after removal of the hydroxyl groups and y is an integer representative of the number of hydroxyl groups on the active-hydrogen compound to which the epoxide is to be transesterified. For example, if the active-hydrogen compound is trimethylolpropane, $R_{13}$ is $CH_3CH_2\text{-}C\text{-}(CH_2\text{-})_3$ and y is 3. If the compound is decanol, $R_{13}$ is $CH_3(CH_2)_8CH_2\text{-}$ and y is 1. If the compound is a polymeric species such as a styrene/allyl alcohol copolymer, $R_{13}$ has the following structure:

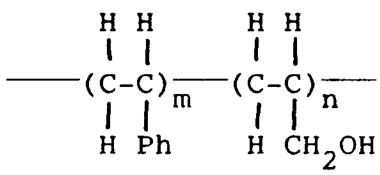

wherein Ph represents a phenyl group, m represents the number of styrene units and n the number of allyl alcohol units in the copolymer, and y is equal to n. When y is greater than one, if desired, not all of the hydroxyl groups on the active-hydrogen compound need be transesterified with epoxide compounds. In this case, the reaction is represented as follows:

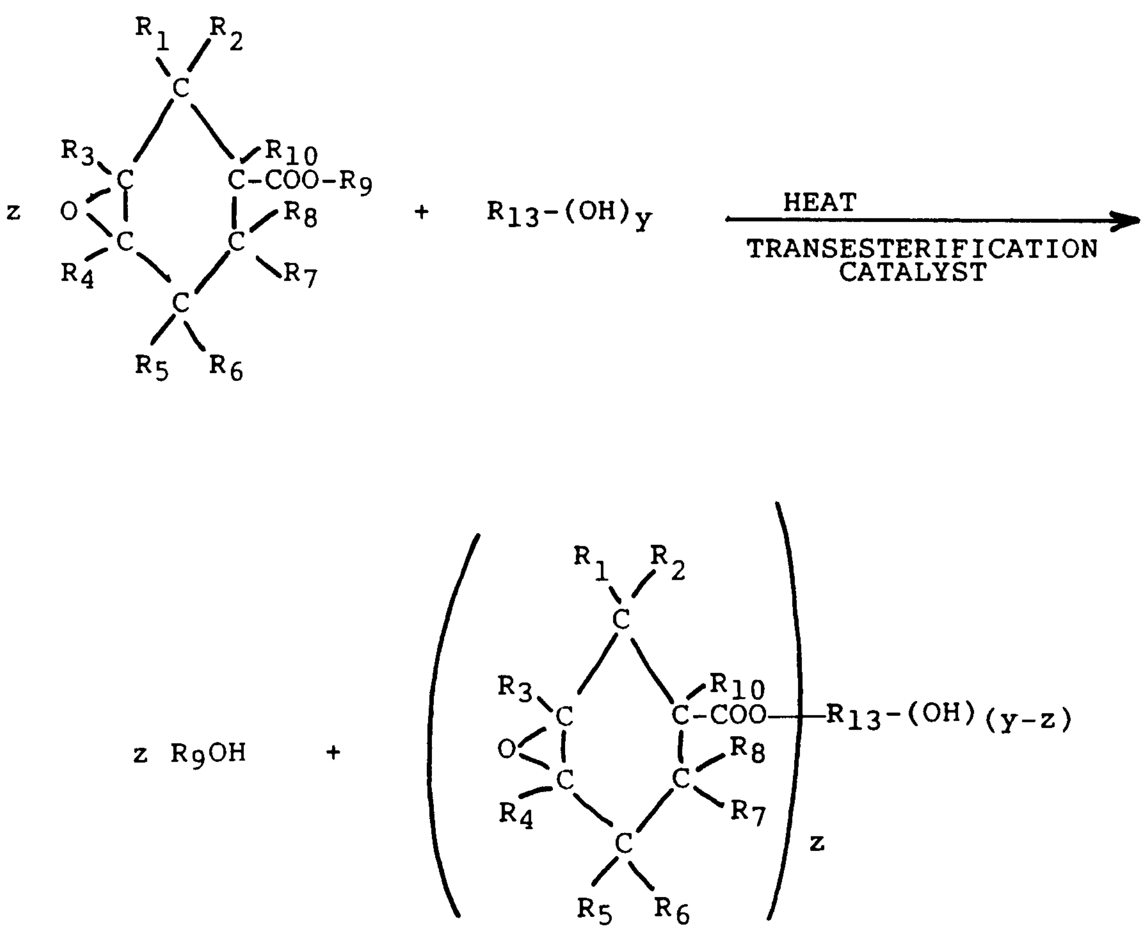


wherein z is less than y. Such compounds, as well as those described elsewhere in the specification, with residual hydroxyl groups can be particularly useful for homopolymerization through reaction of the hydroxyl groups with epoxide groups, especially in the presence of suitable catalysts such as triflic acid and its salts such as diethylammonium triflate, sulfonium salts, tin alkanoates, and the like; for attachment of the compounds to other molecules through the hydroxyl groups; for improved adhesion due to the polarity of the hydroxyl groups; for preparation of urethane derivatives of various types, including moisture-curable derivatives and crosslinked systems; and the like.

When $R_8$ is $-COOR_9'$, the transesterification reaction may be depicted by the following reaction scheme:

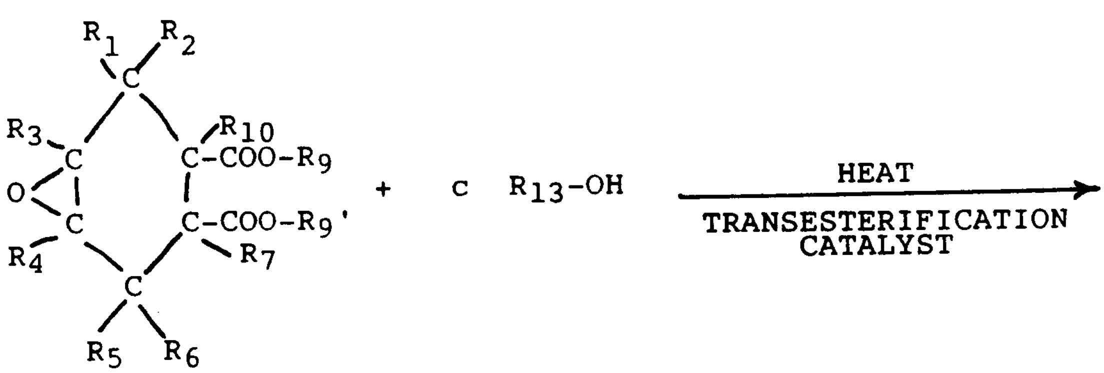

a $R_9OH$ + b $R_9'OH$ +

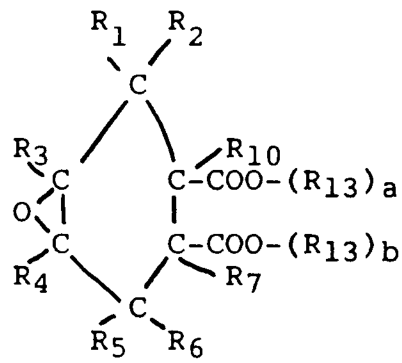

wherein a and b are from zero to one but with the proviso that at least one of a or b not be zero, c = (a + b) is from about 0.1 to about 2, preferably from about 0.5 to about 2, when a monofunctional active-hydrogen compound is used.

When a difunctional active-hydrogen compound is used as in the following reaction:

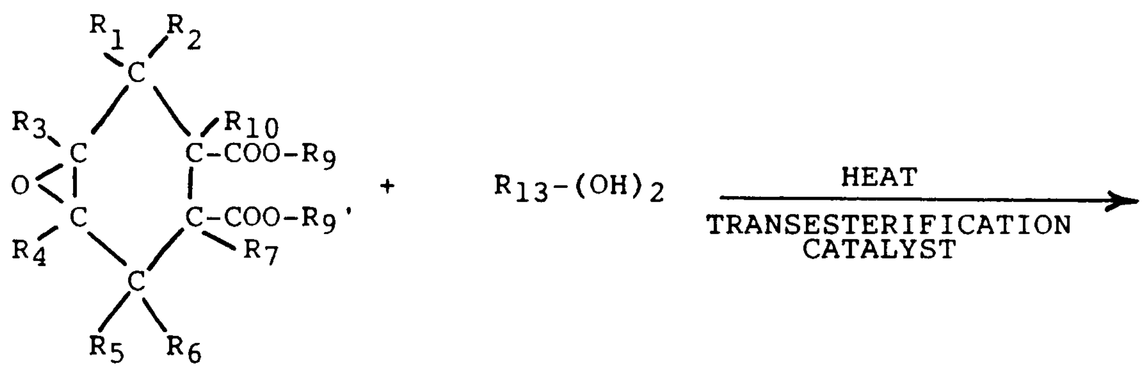


it is understood that a variety of compounds can be obtained, illustrative of which are:

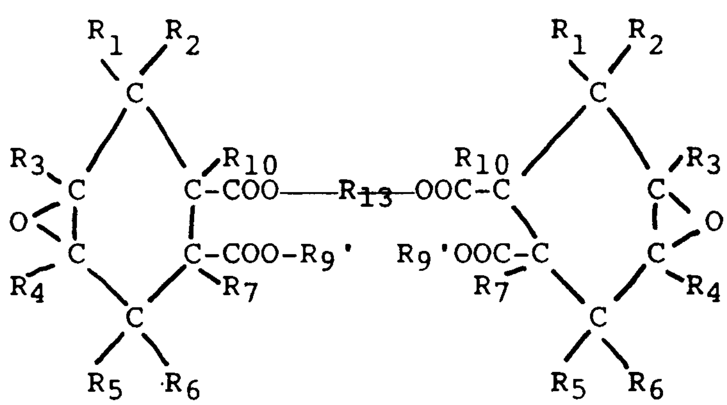

,

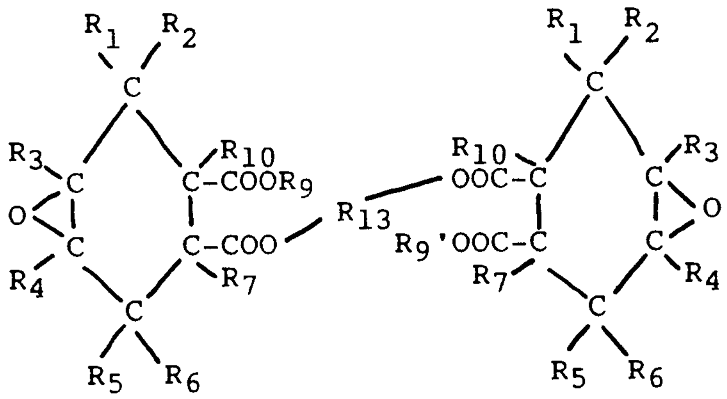

,

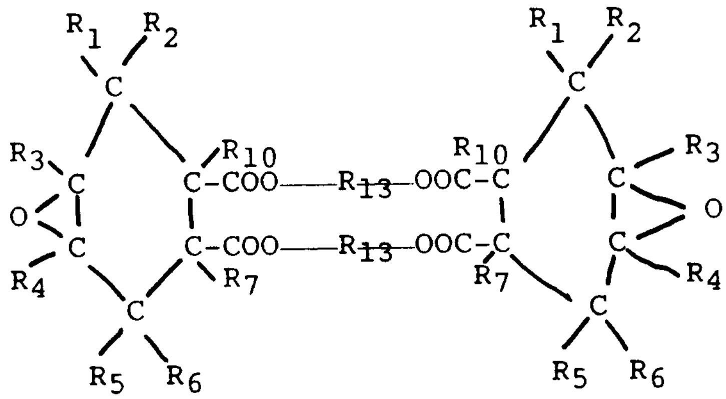

,

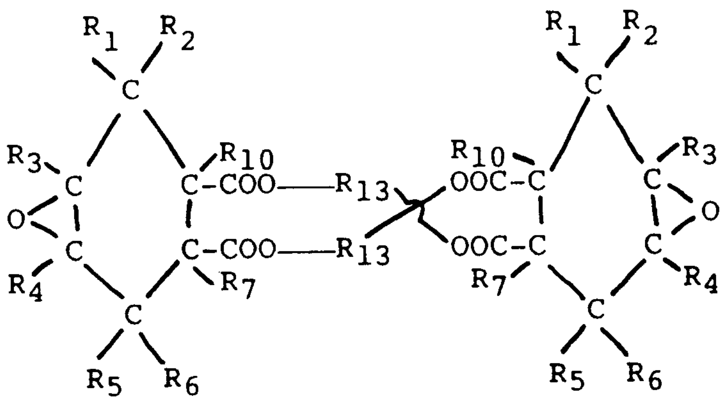

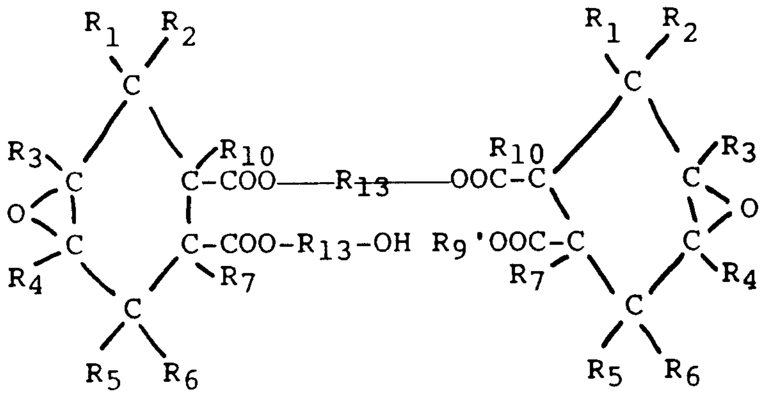

,

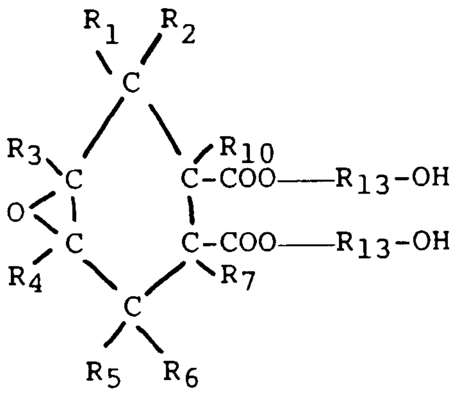

,

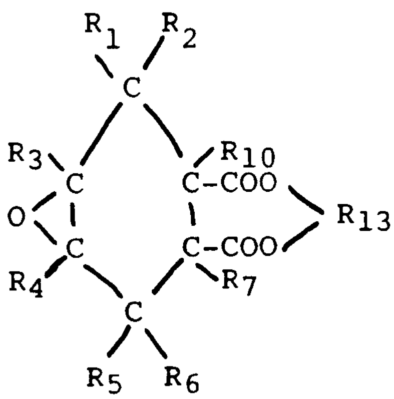

,

and the like. It is further understood that when a mixture of $R_{13}$-OH and $R_{13}$-$(OH)_2$ is used, another variety of compounds can be obtained, illustrative of which are:

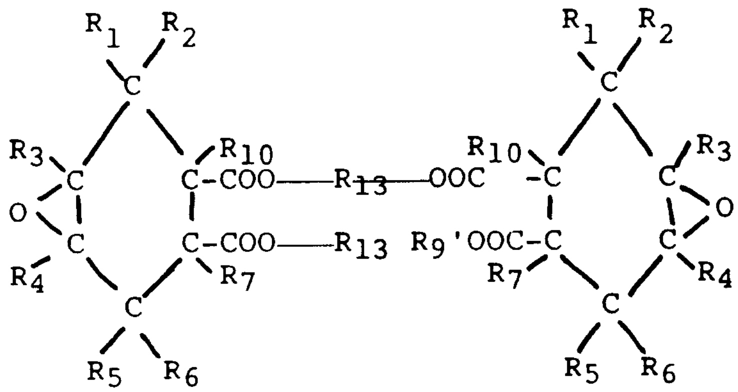

,

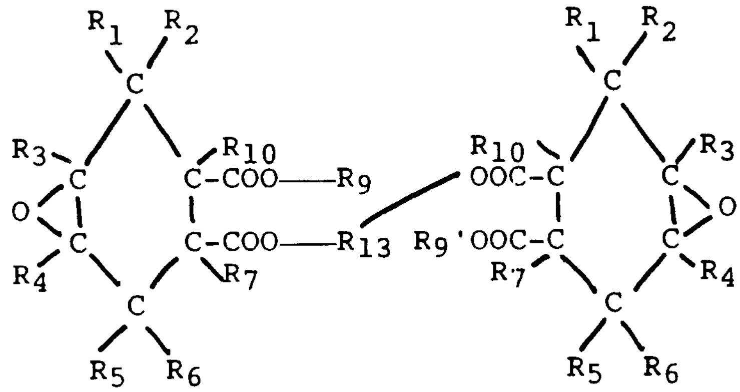

,

and the like.

It is to be still further understood that when a difunctional or higher functionality active-hydrogen compound, preferably a difunctional active-hydrogen compound, is used as in the following reaction, which is exemplified with a difunctional active-hydrogen compound,

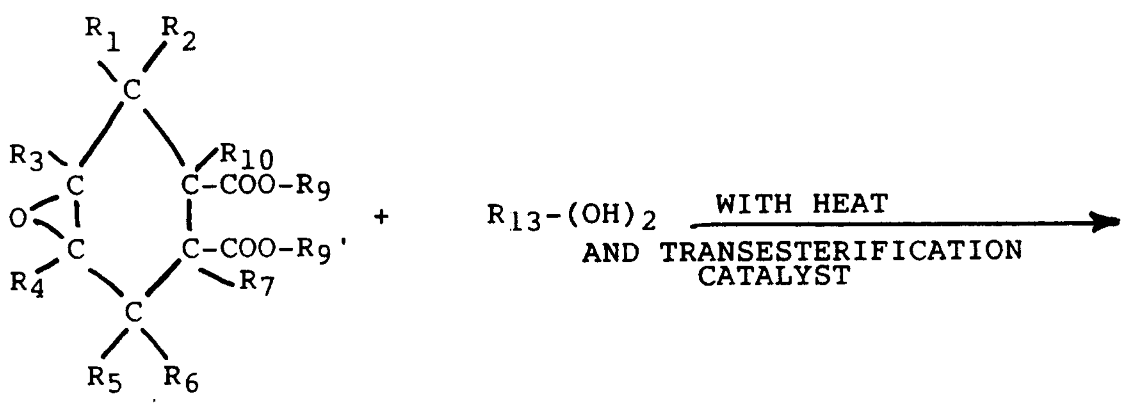


oligomeric and/or polymeric products can be obtained. In addition, it should be realized that average compounds with various end groups can be obtained. Illustrative of the oligomeric and/or polymeric products that can be obtained are the following:

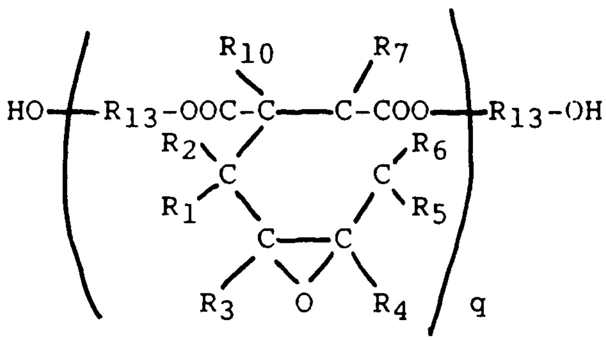

when q molecules of the 3,4-epoxycyclohexane dicarboxylate and (q + 1) molecules of the HO-$R_{13}$-OH diol are used;

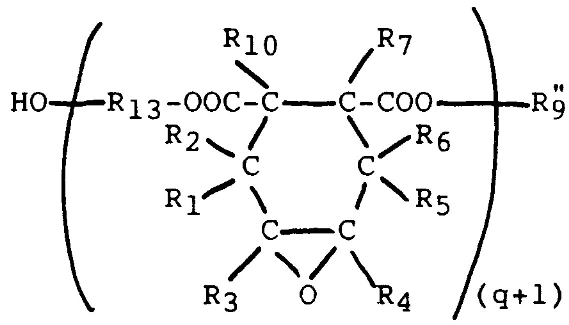

when (q + 1) molecules of the 3,4-epoxycyclohexane dicarboxylate and (q + 1) molecules of the HO-$R_{13}$-OH diol are used and wherein $R_9''$ can be either $R_9$ or $R_9'$ ;

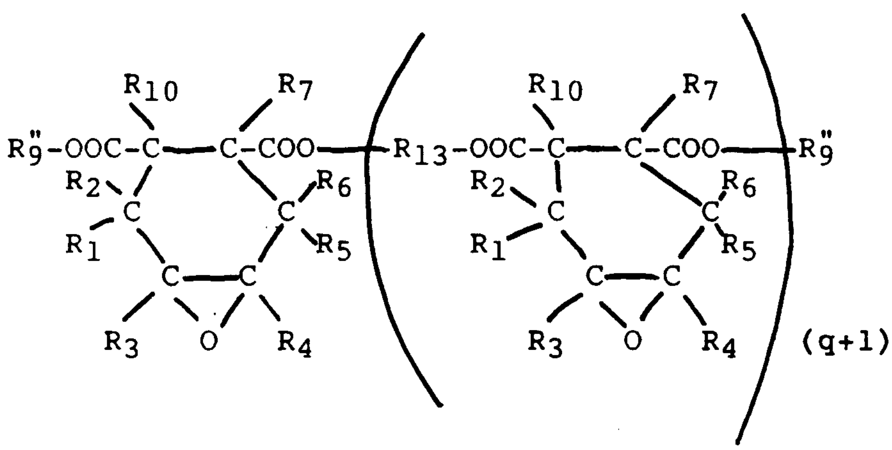

when (q + 2) molecules of the 3,4-epoxycyclohexane dicarboxyate and (q + 1) molecules of the HO-$R_{13}$-OH diol are used; and the like. In addition, ring structures, such as the following

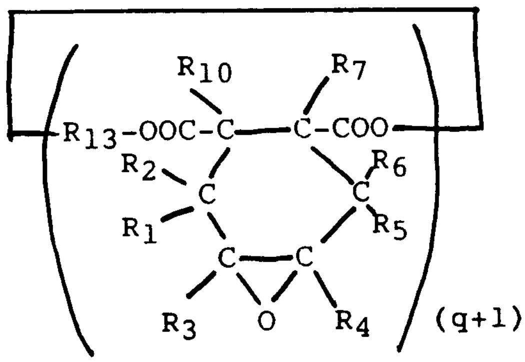

in which no end group exists, can be obtained. It is also understood that mixtures of $R_{13}(OH)_n$ compounds can be used, that $R_{13}$-OH compounds can be used as chain stoppers for control of molecular weight, and that $R_{13}(OH)_3$ and higher functionality active-hydrogen compounds can be used to introduce branching into the oligomeric or polymeric compounds.

In a particular embodiment of this invention, it is possible to prepare unsaturated epoxide products, illustrative of which is bis(tetrahydrobenzyl) 4,5-epoxycyclohexane-1,2-dicarboxylate,

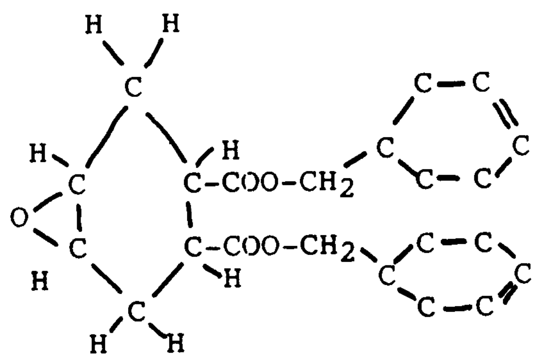

,

which can be prepared by the transesterification of diethyl 4,5-epoxycyclohexane-1,2-dicarboxylate and 3-cyclohexene-1- methanol. This product is useful as an acid scavenger, an intermediate to prepare other compounds, a component of coating, adhesive, ink, or sealant formulations, and the like. If desired, the compound can be reacted with an oxidizing agent such as peracetic or perpropionic acid to form a very reactive triepoxide with the structural formula:

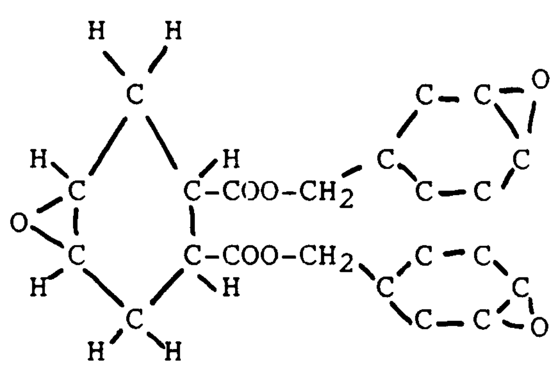

.

It is important to note that for the transesterification process to take place in an efficient manner when a distillation or other vaporization process is used, the molecular weight and/or boiling point of $R_9$-OH must be less than that of $R_{13}$-$(OH)_y$. For example, if $R_9$-OH is ethanol, $R_{13}$-$(OH)_y$ can be propanol, butanols, higher

alcohols, glycerol, glycol, polyol, or the like. Alternately, the $R_{13}$-$(OH)_y$ be present in a large excess.

The mixture of reactants can optionally be dissolved or suspended in an inert solvent for the purpose of facilitating the reaction, reducing the viscosity, or to facilitate handling. Illustrative of the inert solvents are benzene, toluene, xylene, ethyl benzene, trichloroethane, methyl isobutyl ketone, heptanone, methyl amyl ketone, hexane, heptane, and the like.

Suitable reactors for conducting the process of this invention are known to those skilled in the art of organic synthesis and manufacture. These reactors can range from small-scale, laboratory glassware to large, commercial- size manufacturing reactors that are appropriately equipped with control and monitoring devices. The reaction can be conducted in batch, semibatch, or continuous processes and reactors.

The method for operating the process of this invention is the following.

1. A lower-alkyl epoxycyclohexane compound of the form described above is mixed with an active hydrogen compound and a transesterification catalyst in a suitable reactor. The reactor is equipped with a mixing device, a means of heating, cooling, and measuring temperature, and a system for removal of alcohol that forms during the transesterification process.
2. Optionally, an inert solvent may be introduced into the system.
3. The mixture of ingredients is then heated to a temperature of about 50°C to about 250°C and held at this temperature for about 60 minutes to about eight hours under a pressure of about 0.1 mmHg to about 760 mmHg. Preferably, the mixture of reactants is heated to 70°C to 200°C and held at this temperature for about one hour to about 6 hours under a pressure of about 100 mmHg to 760 mmHg.
4. During the reaction, the low molecular weight alcohol of 6 or less carbon atoms formed by the transesterification reaction is removed by either
   (a) sparging of an inert gas through or flowing an inert gas stream over the reacting mixture and removal of alcohol from the flowing gas stream by a condensation system, or
   (b) removal of the alcohol vaporized by a distillation system.
   (c) removal of the alcohol as an azeotrope with an inert solvent, or
   (d) removal of the alcohol by simple distillation at atmospheric or reduced pressure, or
   (e) any combination of 4.(a) through 4.(d), above.

   The process can be monitored by the amount of alcohol removed.
5. The product is either used as a residue product or is washed with water or water saturated with sodium chloride one or more times to remove catalyst residues.
6. The product can then be dried over an inert salt such as magnesium sulfate to remove residual water and then filtered to remove the desiccant and any other suspended matter or, the following step can be employed.
7. The final product can be further purified by vacuum distillation of any residual alcohol that was generated by the transesterification process, wash water, or other impurity, and then used as a residue product or purified further to
   (a) remove any remaining impurities,
   (b) remove any starting epoxide or hydroxyl-containing compound, or
   (c) divide the product into various products, such as monoepoxide, diepoxide, triepoxide, etc.

The cycloaliphatic epoxides of this invention alone or in combination with a variety of other ingredients, such as hydroxyl-containing compounds, other cycloaliphatic epoxides and glycidyl epoxides, acrylates, and so on, can be reacted together to form derivatives, additives, coatings, inks, adhesives, sealants, molded objects, films, powders, and the like. The reactions involved in obtaining these end use products can be carried out by means of actinic radiation, such as ultraviolet light when suitable photoinitiators are employed at essentially ambient conditions, or by means of thermal energy when suitable free or blocked initiators or catalysts are employed, or the like. The compounds can also be used as acid scavengers in poly(vinyl chloride)-containing inks, poly(vinyl chloride) plastisols and organosols, as well as other end uses that require removal of acid. The compounds also can be used as intermediates to form novel compounds in a variety of fields including pharmaceutical compounds, additives for plastics, and the like.

The following examples are illustrative of the present invention and are not intended as a limitation on the scope thereof.

## EXAMPLES

Example 1. Preparation of ethyl 3,4-epoxycyclo- hexanecarboxylate for use in subsequent transesterifications.

Ethyl 3-cyclohexenecarboxylate (125 grams) was placed in a 1000-mL, 3-neck, glass reaction flask equipped with a 500-mL addition funnel, a serum stopper sampling port, mechanical stirrer, and a

thermowatch-equipped thermometer. The addition funnel was charged with 316.9 grams of a 21.42% solution of peracetic acid in ethyl acetate. The contents of the reaction flask were heated, with stirring, to 40-45 ° C by use of the thermowatch and an automatic lab-jack/ice-water bath/heating gun arrangement. Then temperature regulation was achieved in this temperature range, the peracetic acid solution was added to the reaction flask at an average rate of about 5.6 grams per minute (57-minute addition time). When the peracetic acid addition was completed, the addition funnel was flushed with about 10 mL of ethyl acetate. The reaction mixture was held at 40-42 ° C until the peracetic acid concentration was found by titration to be constant at 3.1% which required about a 2-hour reaction time. Chromatographic analysis of the reaction mixture indicated virtually no trace of starting ethyl 3-cyclohexenecarboxylate indicating quantitative conversion to ethyl 3,4-epoxycyclohexanecarboxylate.

The reaction product mixture was placed in a single-neck, one-liter, glass flask and the mixture was concentrated by rotary evaporation at 22 ° C and aspiration vacuum. A dry ice condenser was used to collect condensate, and 156.87 grams were collected. The crude product was then diluted with 137.9 grams of heptane, and this solution was transferred to a one-liter separatory funnel. It was washed with 350 grams of water. This washing was repeated three more times. Titration with 0.1 N KOH to a bromothymol blue endpoint indicated that the fourth was contained 0.09% acetic acid. the organic phase was then transferred to a one-liter, single-neck, glass flask containing a magnetic stir bar and equipped with a 14/20 Virgeaus column, 14/20 short-path distillation head, and a 250-mL collecting flask. The overhead lights (heptane, water, residual ethyl acetate, and acetic acid) were removed by vacuum distillation. The collecting flask was cooled in a dry ice/acetate bath. Both the lights and the product fraction were analyzed by chromatography. The first overhead fraction distilled at 45-55 ° C and 0.28-82 mmHg. this fraction contained only lights and a trace of product. The second fraction distilled at 55-61 ° C and 0.05-0.075 mmHg and weighed 83.1 grams. It consisted of greater than 99% product, ethyl 3,4-epoxycyclohexanecarboxylate, with less than 800 parts-per-million starting ethyl 3-cyclohexenecarboxylate.

Example 2. Preparation of butyl 3,4-epoxycyclohexanecarboxylate by a transesterification with $NaHCO_3$ catalyst.

Seventeen grams of ethyl 3,4-epoxycyclohexanecarboxylate prepared in Example 1, 22 grams of n-butanol, and 0.4 gram of sodium bicarbonate ($NaHCO_3$) were placed in a glass reaction flask equipped with a thermometer, a stirrer, and a nitrogen inlet and outlet. The reaction mixture was heated to 124 ° C and held at this temperature while removing the ethanol produced by the transesterification reaction until no more ethanol was produced. A yield of 71% was obtained. Gas chromatographic analysis indicated the resulting product was more than 70% butyl 3,4-epoxycyclohexanecarboxylate and about 20% unreacted ethyl 3,4-epoxycyclohexanecarboxylate.

Example 3. Preparation of the bis-3,4-epoxycyclohexanecarboxylate ester of neopentyl glycol and the corresponding monoester of neopentyl glycol by transesterification.

Six grams (35 mmol) of ethyl 3,4-epoxycyclohexanecarboxylate, 1.0 grams (9.6 mmols) of neopentyl glycol, 4 grams toluene, and 0.90 gram (0.926 mmol) of potassium thiocyanate were placed in a glass reactor equipped with a stirrer, a distillation/condensation apparatus, and temperature measuring device and heated to 140 ° C with stirring and held at about this temperature for 1.5 hours. Ethanol/toluene distillate was collected at a distillation-head temperature of 62-72 ° C. The reactor contents were cooled, and the reaction mixture was diluted with an equal volume of toluene and then washed three times with water. The toluene solution was then dried over magnesium sulfate, filtered, and the toluene removed by vacuum transfer distillation over a two-hour period at room temperature and reduced pressure that ranged as low as less than 0.10 mmHg. The vacuum was maintained for about 15 minutes after distillation of the toluene ceased. Analysis by nuclear magnetic resonance indicated an 87% yield of bis-3,4-epoxycyclohexanecarboxylate ester of neopentyl glycol that has the following theoretical structure:

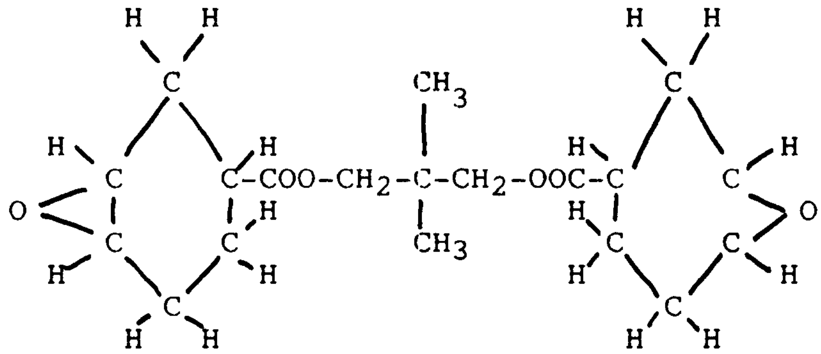

and 13% was the monoester--3,4-epoxycyclohexanecarboxylate monoester of neopentyl glycol--that has the following theoretical structure.

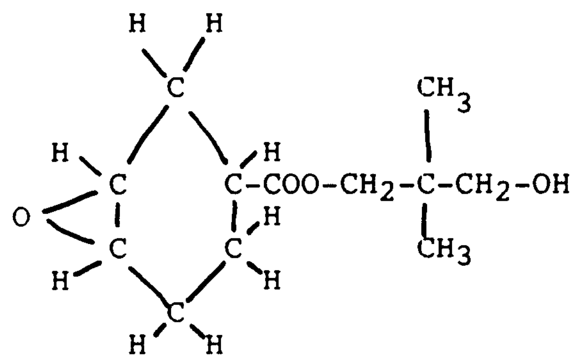

Example 4. Preparation of tris(3,4-epoxycyclohexanecarboxylate) ester of trimethylolpropane.

Thirty-nine grams (291 mmols) of trimethylolpropane (2-ethyl-2-(hydroxymethyl)-1,3-propanediol) and about 140 mL of toluene were placed in a glass reactor equipped with a stirrer, a distillation/condensation apparatus, and temperature measuring device. The mixture was heated to reflux and adventitious water was removed as an azeotrope with toluene. The reaction system was allowed to cool, and 300 grams (1.77 mols) of ethyl 3,4-epoxycyclohexanecarbox- ylate and 2.70 grams (27.8 mmols) of potassium thiocyanate were added. The reaction mixture was heated to 125°C with stirring and held at about this temperature for two hours during which time 173 mL of distillate was collected at a distillation-head temperature of 97-112°C. Then, an additional 100 mL of fresh toluene were added and the distillation was continued for -a sufficient time to remove an additional 82 mL of distillate. The reaction mixture was allowed to cool, was diluted with an equal volume of toluene, and was washed twice with water and once with saturated aqueous sodium chloride. The solution was then dried over magnesium sulfate, filtered to remove the magnesium sulfate, and the toluene was removed by rotary evaporation at temperatures up to 75°C and aspirator vacuum. The product was then stripped under high vacuum by bulb-to-bulb distillation (less than 0.1 mmHg) to remove any remaining toluene (42 grams) and to recover excess ethyl 3,4-epoxycyclohexanecarboxylate, which can be reused. The ethyl 3,4-epoxycyclohexanecarboxylate was collected at 0.3 to 1.7 mmHg and 56-79°C. Complete removal of the excess ethyl 3,4-epoxycyclohexanecarboxylate was confirmed by chromatographic analysis. Analysis of the product indicated that it contained 98 mol percent of the expected triepoxide product and 2 mol percent of the diepoxide, monomethylol of trimethylolpropane product 2-ethyl-2-hydroxymethyl-1,3-propanediol bis(3,4-epoxycyclohexanecarboxylate).

Example 5. Preparation of the hydroxyethyl methacrylate ester of 3,4-epoxycyclohexanecarboxylate and the ethylene glycol ester of 3,4-epoxycyclohexanecarboxylate) .

Ten grams (58.8 mmols) of ethyl 3,4-epoxycyclohexanecarboxylate, 3.21 grams (24.7 mmols) of 2-hydroxyethyl methacrylate, 0.090 gram (0.926 mmol) of potassium thiocyanate, 0.035 gram (0.176 mmol) of phenothiazine and 10.0 grams of toluene were charged to a glass reactor equipped with a stirrer, a distillation/condensation apparatus, and temperature measuring device and heated to 169°C and held at about temperature for 1.5 hours. During this time about 10 milliliters of an ethanol/toluene distillate were collected at a distillation-head temperature of 65-93°C. The reaction mixture was then cooled, diluted with

an equal volume of toluene, and washed once with a saturated aqueous solution of sodium chloride and twice with water. The product was then dried over magnesium sulfate and filtered to remove the magnesium sulfate and any other suspended matter. The dried product was then placed in a rotary evaporator and toluene plus any other volatiles were removed at a temperature of up to 70 ° C under aspirator vacuum. Any remaining toluene and ethyl 3,4-epoxycyclohexanecarboxylate were removed by vacuum distillation at reduced pressures as low as 0.01 mmHg and a temperature of 95 ° C. After distillation ceased, the vacuum was maintained for 30 minutes. Product analysis by gas chromatography indicated that 86% of the theoretical amount of ethyl 3,4-epoxycyclohexanecarboxylate had reacted. Also, 65% of the product was the desired monoepoxide acrylate that has the following theoretical structure

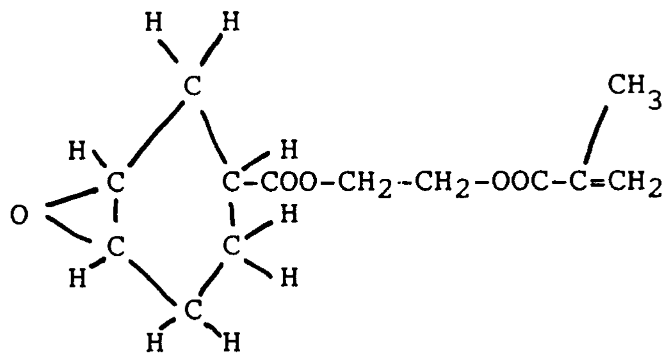

and 35% of the product was a diepoxide product that appeared to be the adduct of ethylene glycol and two moles of ethyl 3,4-epoxycyclohexanecarboxylate which would have the following structure, if the supposition regarding structure is correct:

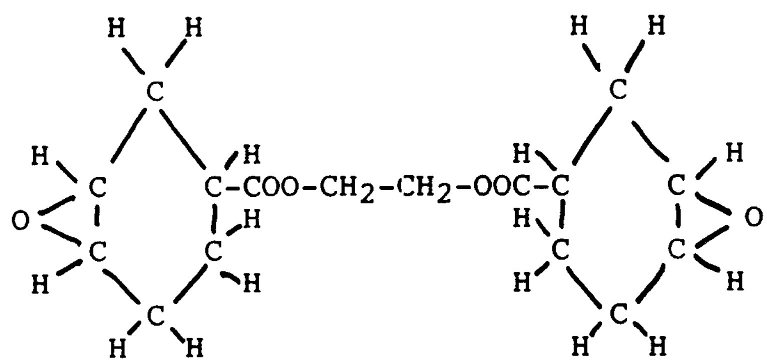

Analysis by nuclear magnetic resonance indicated the product mixture contained 66% of the desired monoepoxide acrylate depicted above and 34% of the side-product diepoxide.

The product mixture is used to prepare coatings, inks, adhesives and sealants by adding onium salt photoinitiators and/or free radical-type photoinitiators and cured by exposure to ultraviolet light. The product mixture is also used to prepare coatings, inks, adhesives, and sealants by adding thermally activatable cationic initiators/catalysts such as triflic acid salts and curing at temperatures of from about 25 ° C to 200 ° C.

Example 6. Preparation of the bis-3,4-epoxycyclohexanecarboxylate ester of 1,4-cyclohexanediol and the corresponding monoester of 1,4-cyclohexanediol by transesterification.

Six grams (35 mmols) of ethyl 3,4-epoxycyclohexanecarboxylate, 1.1 grams (9.6 mmols) of 1,4-cyclohexanediol, 0.090 gram (0.926 mmol) of potassium thiocyanate, and 4.0 grams of toluene were placed in a glass reactor equipped with a stirrer, a distillation/condensation apparatus, and temperature measuring device and heated to 165 ° C while stirring. The transesterification reaction was allowed to proceed for 6.7 hours during which time the reaction temperature varied from 165 ° C to 185 ° C. During this time 3 milliliters of distillate were collected at a distillation-head temperature of 78-89 ° C. The product mixture was then cooled, diluted with an equal volume of toluene and washed once with a saturated aqueous solution of sodium chloride and twice with water. The mixture was then dried over magnesium sulfate, filtered, and the

toluene was removed by rotary evaporation at temperature up to 70 °C under aspirator vacuum. Gas chromatographic analysis of the distillate indicated that 100% of the theoretical amount of ethanol had been obtained and of the product indicated that a mixture containing 55% of a diepoxide with the following theoretical structure:

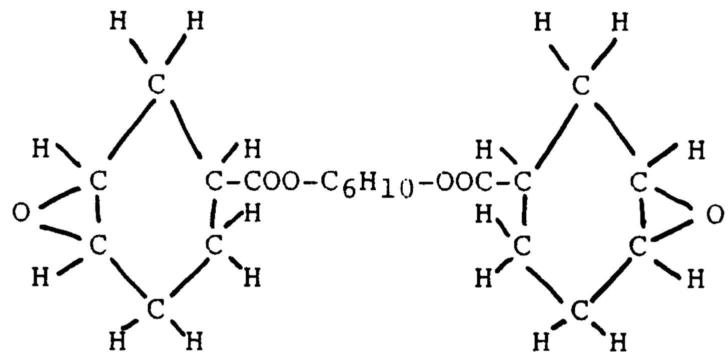

and 45% of the monoepoxide with the following theoretical structure:

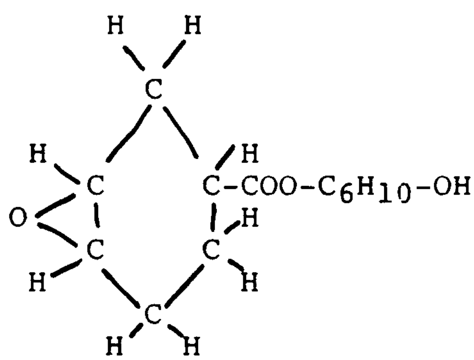

had been obtained.

Similar products are prepared by substituting 1,2- cyclohexanediol, 1,3-cyclohexanediol, or 1,2-cyclohexanedimethanol, or 1,4-cyclohexanedimethanol for the 1,4-cyclohexanediol used above and carrying out the transesterification is the same manner.

Example 7. This example describes a transesterification between trimethylolpropane and methyl 3,4-epoxycyclohexanecarboxylate in which the final product has on the average about one epoxide group and two hydroxyl groups.

Seventy milliliters of toluene and 8.93 grams (66.7 mmols) of trimethylolpropane (2-ethyl-2-(hydroxymethyl)-1,3-propanediol) are placed in a glass reactor equipped with a stirrer, a distillation/condensation apparatus, and temperature measuring device. The mixture is heated to reflux and adventitious water is removed as an azeotrope with toluene. The reaction system is allowed to cool, and 11.2 grams (67 mmols) of ethyl 3,4-epoxycyclohexanecarboxylate and 0.2 gram of potassium thiocyanate are added. The reaction mixture is heated to 150 °C with stirring and held at about this temperature for four hours during which time methanol/toluene distillate is collected. The reaction mixture is allowed to cool, diluted with an equal volume of toluene, and washed twice with water and once with saturated aqueous sodium chloride. Residual water and toluene are removed by rotary evaporation at a temperature of 80 °C and aspirator vacuum. The resulting product is used in a vinyl chloride copolymer/polyurethane coating system as an acid scavenger.

Example 8. This example describes a transesterification process between ditrimethylolpropane and ethyl 3,4-epoxycyclohexanecarboxylate.

4.1 grams of toluene, 2.45 grams (9.78 mmols) of ditrimethylolpropane, 13.4 grams (78.8 mmol) of ethyl 3,4- epoxycyclohexanecarboxylate, and 0.0930 gram (0.957 mmol) of potassium thiocyanate were placed in a glass reactor equipped with a stirrer, a distillation/condensation apparatus, and temperature measuring device. The stirred mixture under a static blanket of argon was heated to 142 °C over a one-hour period.

Distillate was collected over a 1.25-hour period at a reaction temperature of 132-142 °C and a head temperature of 53-75 °C. The reaction product was cooled, diluted with an equal volume of toluene, and washed twice with water and once with aqueous saturated sodium chloride. The resulting product was dried over magnesium sulfate, filtered, and the toluene removed with a rotary evaporator at temperatures up to 70 °C and aspirator vacuum. Nuclear magnetic resonnance analysis of the product indicated that it consisted of 42.5% tetraepoxide, 39.0% triepoxide monomethylol, 16.3% diepoxide dimethylol, and 2.1% monoepoxide trimethylol. Eighty and one-half percent of the methylol groups had been esterified.

Example 9. 4.04 grams of toluene, 1.01 grams (9.6 mmols) of neopentyl glycol, 6.03 grams (35.2 mmols) of ethyl 3,4-epoxycyclohexanecarboxylate, and 0.0562 gram (1.04 mmols) of sodium methoxide were placed in a glass reactor equipped with a stirrer, a distillation/condensation apparatus, and temperature measuring device. The stirred mixture under a static blanket of argon was heated to 160 °C over about a one-hour period. Distillate was collected over a 6.75-hour period at a reaction temperature of 155-160 °C and a head temperature of 60-72 °C. The reaction product was cooled, diluted with an equal volume of toluene, and washed three times with water, dried over magnesium sulfate, and filtered. Toluene and other volatiles were removed with a rotary evaporator at temperatures up to 70 °C and aspirator vacuum. Chromatographic analysis of the product indicated that it consisted of a mixture of 55% of the expected diepoxide, neopentyl glycol bis(3,4-epoxycyclohexanecarboxylate), and 45% of the monoepoxide monoalcohol.

Example 10. Four grams of toluene, 11.2 grams (100 mmols) of tetrahydrobenzyl alcohol, 6.0 grams (25 mmols) of diethyl 4,5-epoxycyclohexane-1,2-dicarboxylate, and 0.090 gram (0.926 mmol) of potassium thiocyanate were placed in a glass reactor equipped with a stirrer, a distillation/condensation apparatus, and temperature measuring device. The stirred mixture under a static blanket of argon was heated to 188 °C, and distillate was collected over a one-hour period at a reaction temperature of 174-188 °C and a head temperature of 72-100 °C. The reaction product was cooled, diluted with an equal volume of toluene, and washed twice with water and once with aqueous saturated sodium chloride solution, dried over magnesium sulfate, and filtered. Toluene and other volatiles were removed with a rotary evaporator at temperatures up to 70 °C and aspirator vacuum, followed by vacuum-transfer distillation to remove toluene at room temperature at a pressure of 1 mmHg. Chromatographic analysis of the product indicated that it consisted of a mixture of 85 mol percent bis(tetrahydrobenzyl) 4,5-epoxycyclohexane-1,2- dicarboxylate,

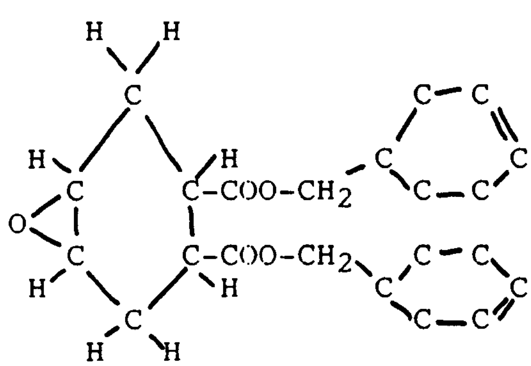

,

and 15 mol percent ethyl tetrahydrobenzyl 4,5-epoxycyclohexane-1,2-carboxylate,

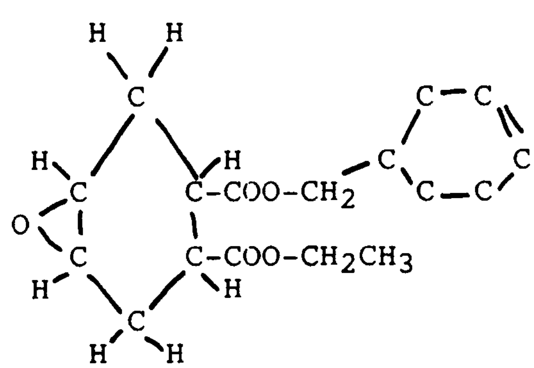

.

These monoepoxides are treated with peracetic acid to form the corresponding triepoxide and diepoxide.

Example 11. Four grams of toluene, 7.83 grams (49.5 mmols) of 1-decanol, 6.0 grams (25 mmols) of diethyl 4,5-epoxycyclohexane-1,2-dicarboxylate, and 0.0920 gram (0.947 mmol) of potassium thiocyanate were placed in a glass reactor equipped with a stirrer, a distillation/condensation apparatus, and temperature measuring device. The stirred mixture under a static blanket of argon was heated to 192 ° C, and distillate was collected over a 1.5-hour period at a reaction temperature of 178-192 ° C and a head temperature of 62-103 ° C. The reaction product was cooled, diluted with an equal volume of toluene, and washed twice with water and once with aqueous saturated sodium chloride solution, dried over magnesium sulfate, and filtered. Toluene and other volatiles were removed with a rotary evaporator at temperatures up to 70 ° C and aspirator vacuum. Nuclear magnetic resonnance analysis of the product indicated a 68:32 mole ratio of didecyl 4,5-epoxycyclohexane-1,2- carboxylate,

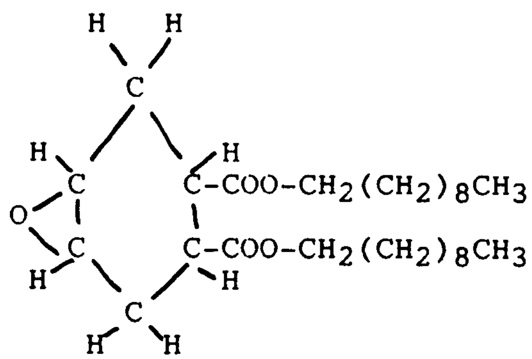

,

and 32 mole percent of a mixture of ethyl, decyl-4,5-epoxycyclohexane-1,2-carboxylate and 1-decyl-2-ethyl 4,5-epoxycyclohexane-1,2-carboxylate.

Example 12. Twenty-five grams of toluene, 3.60 grams (24.9 mmols) of cyclohexane-1,4-dimethanol, 6.0 grams (25 mmols) of diethyl 4,5-epoxycyclohexane-1,2-dicarboxylate, and 0.0982 gram (1.010 mmols) of potassium thiocyanate were placed in a glass reactor equipped with a stirrer, a distillation/condensation apparatus, and temperature measuring device. The stirred mixture under a static blanket of argon was heated to about 150 ° C, and distillate was collected over a three-hour period at a reaction temperature of 140-153 ° C and a head temperature of 75-108 ° C. The reaction product was cooled, diluted with an equal volume of toluene, and washed twice with water and once with aqueous saturated sodium chloride solution, dried over magnesium sulfate, and filtered. Toluene and other volatiles were removed with a rotary evaporator at temperatures up to 70 ° C and aspirator vacuum. An oligomeric product, which chromatographic analysis indicated had an average degree of polymerization of 5.9, was obtained. The product is used formulated with an ultraviolet light-activatable sulfonium salt and cured by exposure to ultraviolet light.

Example 13. Twenty-five grams of toluene, 7.2 grams (50 mmols) of cyclohexane-1,4-dimethanol, 6.0 grams (25 mmols) of diethyl 4,5-epoxycyclohexane-1,2-dicarboxylate, and 0.0976 gram (0.976 mmol) of potassium thiocyanate were placed in a glass reactor equipped with a stirrer, a distillation/condensation apparatus, and temperature measuring device. The stirred mixture under a static blanket of argon was heated to 150 ° C, and 18.1 grams of distillate were collected over a 1.25-hour period at a reaction temperature of 150-155 ° C and a head temperature of 103-110 ° C. The reaction product was cooled, diluted with an equal volume of toluene, and washed twice with water and once with aqueous saturated sodium chloride solution, dried over magnesium sulfate, and filtered. Toluene and other volatiles were removed with a rotary evaporator at temperatures up to 70 ° C and aspirator vacuum. The oligomeric product, which chromatographic analysis indicated had an average degree of polymerization of 2.9, was stored for future use.

Example 14. A mixture of 6.03 grams (35.2 mmols) of ethyl 3,4-epoxycyclohexanecarboxylate, 1.01 grams (9.6 mmols) of neopentyl glycol, and 15 grams of toluene were placed in a glass reactor and heated to reflux with stirring under static argon to azeotropically dry the reaction mixture. Heating was continued for 30 minutes. The reaction mass was cooled to room temperature, and 0.0461 gram (1.095 mmols) of calcium hydride was added. Distillate was collected by means of a short-path distillation apparatus at a head temperature of 100-111 ° C over a 6-hour period. The mixture was allowed to cool and a few milliliters of water were added with stirring to quench the calcium hydride catalyst. The reaction mass was diluted with an equal volume of toluene and washed three times with water. The solution was then dried over

magnesium sulfate, filtered, and the toluene was removed by rotary evaporation under aspirator vacuum. After removal of the toluene, the vacuum and heating were maintained for about 30 minutes after all visible bubbling/distillation ceased. To remove any remaining toluene and some excess ethyl 3,4-epoxycyclohexanecarboxylate, vacuum-transfer distillation was carried out at pressures down to 1 mmHg with the collecting flask cooled in a dry ice/acetone mixture. Chromatographic analysis suggested that the residue product was a mixture of the half-esterified monoepoxide/monoalcohol compound described in Example 3 and excess ethyl 3,4-epoxycyclohexanecarboxylate.

Example 15. A mixture of 6.04 grams (35.3 mmols) of ethyl 3,4-epoxycyclohexanecarboxylate, 1.02 grams (9.7 mmols) of neopentyl glycol, 0.0841 gram (1.023 mmols) of sodium acetate, and 15 grams of toluene were placed in a glass reactor and heated to reflux with stirring under static argon. Distillate was collected overhead by means of a short-path distillation apparatus at a head temperature of 49-58 °C over a 40-minute period. The reaction mixture was cooled to room temperature, diluted with an equal volume of toluene, washed three times with water, dried over magnesium sulfate, and filtered. Toluene was removed by rotary evaporation under aspirator vacuum. After all visible bubbling/distillation ceased, the vacuum and heating was maintained for an additional 30 minutes. To remove any remaining toluene and some excess ethyl 3,4-epoxycyclohex- anecarboxylate, vacuum-transfer distillation was carried out at pressures down to 1 mmHg with the collecting flask cooled in a dry ice/acetone mixture. Chromatographic analysis indicated that the light yellow, residue product consisted of a 99:1 mole ratio of diepoxide, neopentyl glycol bis(3,4-epoxycyclohexanecarboxylate), to the half-esterified intermediate (see Example 3 for structures).

Example 16. A mixture of 6.04 grams (35.3 mmols) of ethyl 3,4-epoxycyclohexanecarboxylate, 1.01 grams (9.6 mmols) of neopentyl glycol, 0.0665 gram (1.022 mmols) of potassium cyanide, and 4 grams of toluene were placed in a glass reactor and heated to reflux with stirring under static argon. Distillate was collected overhead by means of a short-path distillation apparatus at a head temperature of 62-91 °C over a one-hour period. The reaction mixture was cooled to room temperature. Chromatographic analysis of the reaction mixture indicated no trace of the starting glycol. The reaction mixture was diluted with an equal volume of toluene, washed three times with water, dried over magnesium sulfate, and filtered. Toluene was removed by rotary evaporation under aspirator vacuum. After all visible bubbling/distillation ceased, the vacuum and heating was maintained for an additional 30 minutes. To remove any remaining toluene and some excess ethyl 3,4-epoxycyclohexanecarboxylate, vacuum-transfer distillation was carried out at pressures down to 1 mmHg with the collecting flask cooled in a dry ice/acetone mixture. Chromatographic analysis indicated that the light yellow, residue product consisted of a 95:5 mole ratio of diepoxide, neopentyl glycol bis(3,4-epoxycyclohexanecarboxylate), to the half-esterified intermediate (see Example 3 for structures).

Example 17. A mixture of 10.0 grams (59 mmols) of ethyl 3,4-epoxycyclohexanecarboxylate, 1.3 grams (9.7 mmols) of trimethylolpropane, 0.0760 gram (0.926 mmol) of sodium acetate, and 4 grams of toluene were placed in a glass reactor and heated to reflux with stirring under static argon. Distillate was collected overhead by means of a short-path distillation apparatus at a head temperature of 63-76 °C over a 45-minute period. The reaction mixture was cooled to room temperature. Chromatographic analysis of the reaction mixture indicated that the starting trimethylolpropane had been completely consumed. The reaction mixture was diluted with an equal volume of toluene, washed twice with water and once with saturated aqueous sodium chloride, dried over magnesium sulfate, and filtered. Toluene was removed by rotary evaporation under aspirator vacuum. After all visible bubbling/distillation ceased, the vacuum and heating was maintained for an additional 30 minutes. To remove any remaining toluene and excess ethyl 3,4-epoxycyclohexanecarboxylate, vacuum- transfer distillation was carried out at pressures down to 1 mmHg with the collecting flask cooled in a dry ice/acetone mixture. High pressure liquid chromatography indicated that the light yellow, residue product was essentially the expected pure triepoxide, trimethylolpropane tris-(3,4-epoxycyclohexanecarboxylate),.

Example 18. A mixture of 10.05 grams (59.00 mmols) of ethyl 3,4-epoxycyclohexanecarboxylate, 5.26 grams (9.74 mmols) of TONE™ Polyol 1305 (an experimental trifunctional polycaprolactone polyol with an average equivalent weight of 180, available from Union Carbide Chemicals and Plastics Company Inc.), 0.0916 gram (0.942 mmol) of potassium thiocyanate, and 4 grams of toluene were placed in a glass reactor and heated to 164 °C with stirring under static argon. Distillate was collected overhead by means of a short-path distillation apparatus at a head temperature of 45-54 °C over a 2-hour period. The reaction mixture was cooled to room temperature. The reaction mixture was diluted with an equal volume of toluene, washed twice with water and once with saturated aqueous sodium chloride, dried over magnesium sulfate, and filtered. Toluene was removed by rotary evaporation at a temperature up to 70 °C under aspirator vacuum. After all visible bubbling/distillation ceased, the vacuum and heating was maintained for an additional 30 minutes. To remove any remaining toluene and excess ethyl 3,4-epoxycyclohexanecarboxylate, vacuum-

transfer distillation was carried out at pressures down to 1 mmHg with the collecting flask cooled in a dry ice/acetone mixture. Chromatographic analysis of the first distillate indicated that 67 percent of the theoretical amount of ethanol by-product for full substitution had been obtained. The epoxy equivalent weight was determined by titration to be 286, slightly lower than the theoretical value, which indicated essentially complete esterification and that a trifunctional epoxide had been formed.

Example 19. A mixture of 10.05 grams (41.48 mmols) of diethyl 4,5-epoxycyclohexane-1,2-dicarboxylate, 5.24 grams (9.7 mmols) of TONE™ Polyol 1305, 0.090 gram (0.926 mmol) of potassium thiocyanate, and 4 grams of toluene were placed in a glass reactor and heated to 170°C with stirring under static argon. Distillate was collected overhead by means of a short-path distillation apparatus at a head temperature of 79-82°C over a 30-minute period. The reaction mixture was cooled to room temperature. The reaction mixture was diluted with an equal volume of toluene, washed twice with water and once with saturated aqueous sodium chloride, dried over magnesium sulfate, and filtered. Toluene was removed by rotary evaporation at a temperature up to 70°C under aspirator vacuum. Chromatographic analysis of the first distillate indicated that at least 57 percent of the theoretical amount of ethanol by-product had been removed in the distillation. The epoxy equivalent weight was determined by titration to be 389, slightly higher than the theoretical value. Chromatographic analysis also indicated that a significant increase in molecular weight had taken place during the reaction, indicating that oligomerization/polymerization had taken place due to the di- and trifunctional nature of the reactants.

Example 20. A mixture of 13.61 grams (78.76 mmols) of ethyl 3,4-epoxycyclohexanecarboxylate, 2.50 grams (10.0 mmols) of ditrimethylolpropane, 0.1559 gram (1.897 mmols) of sodium acetate, and 6.0 grams of toluene were placed in a glass reactor and heated to 140°C with stirring under static argon. About 2 mL of distillate were collected overhead by means of a short-path distillation apparatus at a head temperature of 62-92°C and a mantle temperature of 140-141°C over a 30-minute period. The reaction mixture was cooled to room temperature. The reaction mixture was diluted with an equal volume of toluene, washed twice with water and once with saturated aqueous sodium chloride, dried over magnesium sulfate, and filtered. Toluene was removed by rotary evaporation at aspirator pressure and 70°C. Residual toluene and ethyl 3,4-epoxycyclohexanecarboxylate were removed by vacuum transfer distillation at a temperature up to 70°C and 1 mmHg. High pressure liquid chromatographic analysis indicated that the light straw-colored product consisted primarily of the tetraepoxide, ditrimethylolpropane tetrakis(3,4-epoxycyclohexanecarboxylate).

Example 21. A mixture of 6.00 grams (35.0 mmols) of ethyl 3,4-epoxycyclohexanecarboxylate, 20.00 grams (222.0 mmols) of 2-ethoxyethanol, and 0.0720 gram (1.03 mmols) of sodium acetate were placed in a glass reactor and heated to 148°C with stirring under static argon. About 2 mL of distillate were collected overhead by means of a short-path distillation apparatus at a head temperature of 58-74°C and a mantle temperature of 148-165°C over a one-hour period. The reaction mixture was cooled to room temperature. The reaction mixture was then vacuum distilled to remove most of the excess 2-ethoxyethanol. The remaining reaction mixture was diluted with 40 mL of toluene, washed once with water and once with saturated aqueous sodium chloride, dried over magnesium sulfate, and filtered. Toluene was removed by rotary evaporation at aspirator pressure and 70°C. Residual 2-ethoxyethanol, toluene and ethyl epoxycyclohexanecarboxylate were removed by vacuum transfer distillation at a temperature up to 90°C and 1 mmHg. Chromatographic analysis indicated that the product contained 60% of the expected monoepoxide, 4% residual ethyl 3,4-epoxycyclohexanecarboxylate, and 36% unidentified products.

Examples 22-39. Illustrative Examples. The following ingredients are placed in a suitable container and well mixed. They are then coated onto metal panels, wood, paper, paperboard, plastic and vinyl-tile substrates with a No. 20 wire-wound rod. the coated substrates are then exposed to a 200 watt-per-inch, medium-pressure-mercury-vapor ultraviolet-light source at speeds ranging from 10 to 100 feet-per-minute. Tack-free coatings with useful properties result.

| EXAMPLE | | | | | | |
|---|---|---|---|---|---|---|
| Ingredients, g | 22 | 23 | 24 | 25 | 26 | 27 |
| Example 3 Product | 97.0 | 43.0 | 10.0 | -- | -- | -- |
| Example 4 Product | -- | -- | -- | 99.0 | 75.0 | 5.0 |
| Example 1 Product | -- | 10.0 | -- | -- | -- | 10.0 |
| 3,4-epoxycyclohexanemethyl 3,4-epoxycyclohexanecarboxylate | -- | 45.0 | 56.0 | -- | -- | 62.0 |
| Trimethylolpropane triacrylate | -- | -- | -- | -- | 23.0 | -- |
| Propylene glycol | -- | -- | -- | -- | -- | 20.0 |
| Aryl sulfonium antimony salt photoinitiator# | 3.0 | 2.0 | -- | 1.0 | 2.0 | 3.0 |
| Aryl sulfonium phosphorous salt photoinitiator## | -- | -- | 4.0 | -- | -- | -- |

# CYRACURE™ UVI-6974, a hexafluoroantimonate aryl sulfonium salt marketed by Union Carbide Corporation.
## CYRACURE™ UVI-6990, a hexafluorophosphate aryl sulfonium salt marketed by Union Carbide Corporation.

| | EXAMPLE | | | | | |
|---|---|---|---|---|---|---|
| Ingredients, g | 28 | 29 | 30 | 31 | 32 | 33 |
| Example 5 Product | 30.0 | -- | -- | -- | -- | -- |
| Example 6 Product | -- | 98.0 | 60.0 | -- | -- | -- |
| Example 8 Product | -- | -- | -- | 98.0 | 35.0 | 10.0 |
| 3,4-epoxycyclohexanemethyl 3,4-epoxycyclohexanecarboxylate | 66.0 | -- | 30.0 | -- | 35.0 | 62.0 |
| Trimethylolpropane triacrylate | -- | -- | 6.0 | -- | 27.0 | -- |
| Poly(tetramethylene oxide) polyol** | -- | -- | -- | -- | -- | 25.0 |
| Aryl sulfonium antimony salt photoinitiator# | -- | 2.0 | 4.0 | 2.0 | 3.0 | 3.0 |
| Aryl sulfonium phosphorous salt photoinitiator# | 4.0 | -- | -- | -- | -- | -- |

*A 1000 average molecular weight, difunctional poly(tetramethylene oxide) polyol marketed by Quaker Chemical as Polymeg™ 1000.
# CYRACURE™ UVI-6974, a hexafluoroantimonate aryl sulfonium salt marketed by Union Carbide Corporation.
## CYRACURE™ UVI-6990, a hexafluorophosphate aryl sulfonium salt marketed by Union Carbide Corporation.

| Ingredients, g | 34 | 35 | 36 | 37 | 38 | 39 |
|---|---|---|---|---|---|---|
| | EXAMPLE | | | | | |
| Example 11 Product | 7.0 | -- | -- | -- | -- | 2.0 |
| Example 13 Product | -- | 98.0 | 25.0 | -- | -- | -- |
| Example 18 Product | -- | -- | -- | 98.0 | 30.0 | 10.0 |
| Example 21 Product | -- | -- | -- | -- | 15.0 | -- |
| 3,4-epoxycyclohexane-methyl 3,4-epoxycyclo-hexanecarboxylate | 80.0 | -- | 55.0 | -- | 35.0 | 85.0 |
| Trimethylolpropane triacrylate | 10.0 | -- | -- | 27.0 | -- | -- |
| Polycaprolactone polyol* | -- | -- | -- | -- | 20.0 | -- |
| Poly(tetramethylene oxide) polyol** | -- | -- | 17.0 | -- | -- | -- |
| Aryl sulfonium antimony salt photoinitiator# | 3.0 | 2.0 | 3.0 | 2.0 | 3.0 | 3.0 |

*A 300 average molecular weight trifunctional polycaprolactone polyol marketed by Union Carbide Corporation under the tradename TONE™ 0305.

**A 1000 average molecular weight, difunctional poly(tetramethylene oxide) polyol marketed by Quaker Chemical as Polymeg™ 1000.

# CYRACURE™ UVI-6974, a hexafluoroantimonate aryl sulfonium salt marketed by Union Carbide Corporation.

Example 40. One hundred grams of the product of Example 10 and 2 grams of CYRACURE™ UVI-6974 are placed in a glass container, well mixed, coated onto a steel or release paper substrate and exposed to a 300 watt-per-inch medium pressure mercury vapor lamp at ten feet-per-minute. Twelve hours after exposure, the resulting polymeric mass is removed by dissolution in ethyl acetate. The solution is then treated with peracetic acid to form a polymeric epoxycyclohexane product. The polymeric epoxide is formulated with onium salt photoinitiators and optionally polyols, other cycloaliphatic epoxides, and surfactants, coated onto metal, plastic, paper, wood and glass substrates, and subjected to ultraviolet light radiation to form useful functional and/or decorative coatings.

Example 41. The one mole of the product of Example 5, 2 moles of styrene, 10 grams of VAZO 52 (azo-based polymerization initiator sold by Du Pont), and 200 grams of methyl isobutyl ketone are placed in a glass reactor equipped with a stirrer, temperature-measuring device, a nitrogen inlet and outlet, and a water-cooled condenser. The mixture is heated to reflux (about 115 °C) and maintained at this temperature for 45 minutes and then the temperature is decreased to 85 °C and held at this temperature for three hours. The product is cooled to room temperature and stored for future use.

Example 42. One-half mole of the product of Example 5, 1 mole of styrene, one mole of t-butyl acrylate, 10 grams of VAZO 52 (azo-based polymerization initiator sold by Du Pont), and 200 grams of methyl isobutyl ketone are placed in a glass reactor equipped with a stirrer, temperature measuring device, and a nitrogen inlet and outlet, and a water-cooled condenser. The mixture is heated to reflux (about 115 °C) and maintained at this temperature for 30 minutes and then the temperature is decreased to 80 °C and held at this temperature for two hours. The resulting product is cooled to room temperature and stored for future

use.

Example 43. Under ultraviolet light-free conditions, the product of Example 42 is formulated with an onium salt photoinitiator, the solvent is removed by spray drying, and the resulting product is used as a powder coating by electrostatically spraying it onto a substrate and heating to melt the powder and then subjecting the film to ultraviolet light. If desired, the spray dried powder may be fluid energy milled to improve its solid-state flow characteristics. Optionally, the product of Example 42 is blended with vinyl chloride copolymers or homopolymers, spray dried, and used as a powder coating with high quality thermal and weathering characteristics.

## Claims

1. A transesterification process in which a mixture of (a) a lower-alkyl epoxycyclohexanecarboxylate comprising the following structure

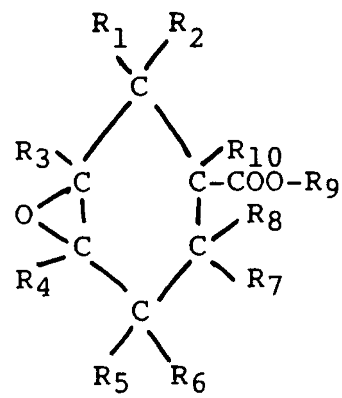

wherein $R_1$ through $R_7$ are the same or different and are hydrogen, phenyl, halogen or unsubstituted or substituted lower alkyl groups of one to about six carbon atoms, $R_8$ is hydrogen, phenyl, halogen, $-COOR_9'$, or unsubstituted or substituted lower alkyl groups of one to about six carbon atoms, $R_9'$ and $R_9$ are the same or different and are lower alkyl of one to about six carbon atoms, and $R_{10}$ is hydrogen or methyl, (b) an active hydrogen-containing compound, (c) a transesterification catalyst comprising a salt of a weak acid, and optionally (d) an inert solvent are heated to a reaction temperature of about 50 ° C to about 250 ° C under a pressure of about 0.1 mmHg to about one atmosphere for a period of time sufficient to cause reaction to take place, and alcohol products are removed, after which time the product is cooled and optionally purified by washing, evaporation, drying, filtration, and/or distillation.

2. A process as in Claim 1 wherein at least one of $R_3$ or $R_4$ is hydrogen.

3. A process as in Claim 1 or 2 wherein $R_9$ and $R_9$'are the same or different and contain one to four carbon atoms.

4. A process as in Claim 1 wherein $R_1$, $R_2$, $R_5$, $R_6$, $R_7$, and $R_8$, as well as one of $R_3$ and $R_4$, are hydrogen and that the other of $R_3$ and $R_4$, are methyl, that $R_9$ and $R_9'$ are the same or different and contain one to four carbon atoms, and $R_{10}$ is hydrogen or methyl.

5. A process as in Claim 1 wherein $R_1$ through $R_8$ are hydrogen, $R_9$ contains one to four carbon atoms, and $R_{10}$ is hydrogen or methyl.

6. A process as in Claim 1 wherein $R_1$ through $R_7$ are hydrogen, $R_8$ is $-COOR_9'$, $R_9$ and $R_9'$ contain one to four carbon atoms, and $R_{10}$ is hydrogen or methyl.

7. A process as in Claim 5 wherein the lower-alkyl epoxycyclohexanecarboxylate is ethyl 3,4-epoxycyclohexanecarboxylate.

8. A process as in Claim 6 wherein the lower-alkyl epoxycyclohexanecarboxylate is diethyl 4,5-epoxycyclohexane-1,2-dicarboxylate.

**9.** A process as in Claim 1 wherein the transesterification catalyst comprises a mixture of salts of weak acids.

**10.** A process as in Claim 1 wherein the transesterification catalyst is sodium bicarbonate; potassium thiocyanate; sodium methoxide; a salt of acetic acid, as sodium acetate;potassium cyanide or calcium hydride.

**11.** A process as in Claim 1 wherein the active hydrogen-containing compound is a compound containing one or more hydroxyl groups.

**12.** A process as in Claim 11 wherein the active hydrogen-containing compound is an alcohol or other monohydroxyl-containing compound.

**13.** A process as in Claim 11 wherein the active hydrogen-containing compound is a glycol or other dihydroxyl-containing compound; a polyol; an oligomeric or polymeric compound; or a mixture of one or more of alcohols, glycols, polyols, oligomers, or polymeric compounds.

**14.** A process as in Claim 12 wherein the active hydrogen-containing compound is hydroxyethylmethacrylate; n-butanol; 2-ethoxyethanol; decanol or tetrahydrobenzyl alcohol.

**15.** A process as in Claim 13 wherein the active hydrogen-containing compound is neopentyl glycol; 1,4-cyclohexane diol; cyclohexane-1,4-di-methanol trimethylolpropane; a polycaprolactone polyol or ditrimethylolpropane.

**16.** A product prepared by the process of Claims 1 to 15.

**17.** A process as in Claim 1 wherein the reaction temperature is from about 70 ° C to about 200 ° C.

**18.** A process of Claim 1 comprising the steps of
(a) mixing a lower-alkyl epoxycyclohexane carboxylate with an active hydrogen compound and a transesterification catalyst comprising one or more salts of one or more weak acids in a suitable reactor,
(b) optionally adding an inert solvent to the mixture in the reactor,
(c) heating the reactants to a temperature sufficient to cause transesterification and to distill over the $R_9OH$ and $R_9'OH$ lower-alkyl alcohol formed from the transesterification process,
(d) optionally diluting or solvent-exchanging the inert solvent with the same or a different inert solvent,
(e) optionally removing by distillation or extraction any inert solvent or other volatiles,
(f) cooling the product.

**19.** A process of Claim 18 in which the mixture of compounds is reacted is heated to 70 ° C to 200 ° C and held at this temperature for about 10 minutes to 40 hours under a pressure of about 0.1 mmHg to 760 mmHg.

**20.** A process of Claim 18 comprising the additional steps of
(a) washing the product one or more times with water, followed with a wash of water saturated with sodium chloride or other salt,
(b) separation of the salt solution from the product by decantation or other separation process,
(c) optionally drying the product with a salt,
(d) optionally filtering the product to remove the drying salt and/or other suspended matter,
(e) further purifying the product by vacuum distillation,
(f) optionally distilling at an elevated temperature to separate the product into various component products.

**21.** A process of Claims 18 or 20 in which the lower-alkyl alcohol is removed by sparging or flowing an inert gas through or over the reacting mixture.

**22.** The process of Claims 18 or 20 in which the lower-alkyl alcohol is removed by condensation of the

vaporized alcohol.

**23.** The cycloaliphatic epoxide products prepared by the process of Claims 18 or 20.

European Patent Office

# PARTIAL EUROPEAN SEARCH REPORT

which under Rule 45 of the European Patent Convention shall be considered, for the purposes of subsequent proceedings, as the European search report

Application Number

EP 91 11 6588

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 57, no. 12, 10th December 1962, columns 15035e-15036e, Columbus, Ohio, US; L.A. MUKHAMEDOVA et al.: "The synthesis and properties of dialkyl epoxytetrahydrophthalates" & NEFTEKHIMIYA 1, No. 1, 88-92(1961) * Abstract * --- | 16,23 | C 07 D 301/00<br>C 07 D 303/40<br>C 08 G 63/91 //<br>C 08 G 59/20 |
| X | CHEMICAL ABSTRACTS, vol. 60, no. 12, 8th June 1964, columns 14455g-14457a, Columbus, Ohio, US; L.A. MUKHAMEDOVA et al.: "Synthesis of some derivates of oxides of esters of delta3-cyclo-hexenecarboxylic acids" & NEFTEKHIMIYA 4(1), 100-5 (1964) * Abstract * --- | 16,23 | |
| X,Y | GB-A- 969 041 (CIBA) * The whole document, particularly examples I and IV * --- -/- | 1-23 | |

TECHNICAL FIELDS SEARCHED (Int. Cl.5)

C 07 D 301/00
C 07 D 303/00

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

The number of already known compounds falling within the scope of claims 16 and 23 is so large that an exhaustive search report for these claims is economically not justified (see Guidelines B-III 2.1).

| Place of search | Date of completion of the search | Examiner |
|---|---|---|
| THE HAGUE | 03-01-1992 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number

EP 91 11 6588

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| Y | US-A-3 070 608 (F.E. KOESTER et al.)<br>* The whole document, particularly column 1, lines 14-21 *<br>--- | 1-23 | |
| D,Y | US-A-4 228 084 (R. ACKERMANN et al.)<br>* The whole document *<br>----- | 1-23 | |
| | | | TECHNICAL FIELDS SEARCHED (Int. Cl.5) |